# EUROPEAN PATENT APPLICATION

(11) **EP 4 194 567 A1**
(43) Date of publication of application: **14.06.2023**
(21) Application number: 21854491.4
(22) Date of filing: 05.08.2021
(51) Int. Cl.: C12Q 1/6886

(54) **PANCREATIC CANCER DIAGNOSTIC COMPOSITION TO BE USED IN BUFFY COAT SAMPLE**

(30) Priority: 06.08.2020 KR 20200098845
(71) Applicant: Huvet Bio, Inc., Seoul 05836 (KR)
(72) Inventor: JEONG, Hyoung Hwa, Seoul 05649 (KR); KIM, Sobin, Seoul 06002 (KR)
(74) Representative: Bates, Philip Ian
(86) International application number: PCT/KR2021/010329
(87) International publication number: WO 2022/031072

(57) **Abstract**

The present invention relates to: a pancreatic cancer diagnostic biomarker comprising one or more selected from the group consisting of Interleukin-28 (IL-28), Interleukin-29 (IL-29) and genes encoding same; a composition for diagnosing pancreatic cancer, comprising a preparation capable of detecting the biomarker; and a pancreatic cancer diagnostic method using the composition, and the present invention is to be used in a blood-derived buffy coat sample.

## Description

### Technical Field

The present disclosure relates to a biomarker for diagnosis of pancreatic cancer, a composition for diagnosis of pancreatic cancer comprising an agent capable of detecting the biomarker, and a method for diagnosing pancreatic cancer using same.

### Background Art

The term "biomarker" generally refers to a measured characteristic which may be used as an indicator of some change caused in an organism by an external factor. Active studies have recently been made to apply biomarkers to the diagnosis of various diseases, such as cancer, nervous system diseases, etc., and to the prediction or monitoring of therapeutic effects of some agents.

The pancreas is a 20-cm-long organ located in the abdomen behind the stomach and functions to secrete pancreatic juice and hormones. The term "pancreatic term" is, for the most part, used to refer to pancreatic adenocarcinoma. Pancreatic cancer is gradually increasing as Western-style diets become more common, and it is known to be found mainly in men, with a high mortality rate. Pancreatic cancer is difficult to detect early because symptoms do not appear well in the early stage. Smoking, coffee, alcohol consumption, meat-oriented eating habits, medical histories such as diabetes, chronic pancreatitis, nonpolyposis colorectal cancer syndrome, etc., and substances such as beta-naphthylamine and benzidine are known to cause pancreatic cancer. Persons with pancreatic cancer do not feel significant symptoms in the early stage, and it is common for symptoms such as pain and weight loss to appear after systemic metastasis has already occurred, so the mortality rate is very high.

Therefore, there is a need for the development of an early diagnosis method for pancreatic cancer.

### Disclosure

### Technical Problem

An aspect provides a biomarker for diagnosis of pancreatic cancer, the biomarker comprising at least one selected from the group consisting of interleukin 28 (IL-28), interleukin 29 (IL-29), and genes therefor. The biomarker may be used for detection or analysis in a blood-derived buffy coat.

Another aspect provides a composition or a kit for diagnosis of pancreatic cancer, the composition or the kit comprising an agent capable of detecting at least one selected from the group consisting of interleukin 28, interleukin 29, and genes coding therefor. The pancreatic cancer diagnosis composition or kit may be applied to a blood-derived buffy coat.

A further aspect provides a method for diagnosing pancreatic cancer or for providing information for diagnosis of pancreatic cancer, the method comprising a step of detecting at least one selected from the group consisting of interleukin 28, interleukin 29, and genes coding therefor in a blood sample isolated from a subject. The blood sample may comprise a blood-derived buffy coat. The detecting step may comprise determining whether at least one selected from the group consisting of interleukin 28, interleukin 29, and genes coding therefor is present or absent in a blood sample and/or measuring a level of at least one selected from the group consisting of interleukin 28, interleukin 29, and genes coding therefor in a blood sample.

A still further aspect provides a use of at least one selected from the group consisting of interleukin 28 (IL-28), interleukin 29 (IL-29), and genes coding therefor or an agent capable of detecting the biomarker for diagnosis of pancreatic cancer and/or for preparation of a pancreatic cancer diagnosing agent. The diagnosis of pancreatic cancer or the detection of the biomarker may be carried out in a blood-derived buffy coat.

### Technical Solution

Below, a detailed description will be given of the present disclosure:

### Diagnosis of Pancreatic Cancer

"Pancreatic cancer", as used herein, is a generic term for tumors that develop in the pancreas and may be exemplified by benign tumors, such as cystic tumors, e.g., serous cystic tumor, mucinous cystic tumor, intraductal papillary mucinous tumor, solid papillary tumor, lymphoepithelial cyst, and cystic teratoma, and malignant tumors such as pancreatic ductal adenocarcinoma, acinar cell carcinoma, and neuroendocrine tumor. The pancreatic cancer that can be diagnosed by the biomarker provided herein may be selected from the tumors given above, for example, malignant tumors, but with no limitation thereto.

As used herein, the term "diagnosis" is intended to encompass determining the susceptibility of a subject to a certain disease or disorder, determining whether a subject is affected with a certain or disorder, determining the prognosis of a subject affected with a certain or disorder (e.g., identifying pre-metastatic or metastatic states of cancer, determining cancer stages or the responsiveness of cancer to treatment), or therametrics (e.g., monitoring the state of a subject to provide information on therapeutic efficacy).

Herein, the diagnosis of pancreatic cancer may mean the determination of the onset or the onset plausibility (risk) of pancreatic cancer.

### Biomarker for Diagnosis of Pancreatic Cancer

Provided herein interleukin 28 (IL-28), interleukin 29 (IL-29), or a combination thereof as a biomarker for diagnosis of pancreatic cancer. The biomarker herein may mean a protein and/or a gene coding therefor.

In an embodiment, the biomarker for diagnosis of pancreatic cancer may be interleukin 28. In another embodiment, the biomarker for diagnosis of pancreatic cancer may be a combination of interleukin 28 and interleukin 29. In a further embodiment, the biomarker for diagnosis of pancreatic cancer may be interleukin 29.

The biomarker is present at a significantly higher expression level in a sample from a pancreatic cancer patient than that from a normal control.

"Interleukin 29" (IL-29), also called interferon lambda-1, is a protein encoded by the IL-19 gene located on chromosome 19. The protein is a member of the helical cytokine family and is a type III interferon. Herein, IL-29 may be derived from humans, for example, NCBI Accession No. NP_742152.1 (SEQ ID NO: 2), but with no limitations thereto. A gene coding for IL-29 may be derived from humans, for example, may be represented by NCBI Accession No. NM_172140.2, but is not limited thereto.

"Interleukin 28" (IL-28) is a cytokine that comes in two isoforms, IL-28A (also called interferon lambda-2 (IFNL2)) and IL-28B (also called interferon lambda-3 (IFNL3)), and plays a role in immune defense against viruses. Herein, IL-28A and IL-28B may be derived from humans. For example, IL-28A and IL-28B may be represented by NCBI Accession No. NP_742150.1 (SEQ ID NO: 3) (NM_172138.2) and NCBI Accession No. NP_742151.2 (SEQ ID NO: 3) (NM_172139.4), respectively, but with no limitations thereto. Genes coding for IL-28A and IL-28B may be derived from humans and may be, for example, represented by NCBI Accession No. NM_172138.2 (IL-28A) and NCBI Accession No. NM_172139.4 (IL-28B), respectively, but with no limitations thereto. As here herein, IL-28 may mean IL-28A, IL-28B, or both of them, for example, IL-28A.

Details of the biomarkers are given in Table 1, below.

**TABLE 1**

| Marker | Gene Accession No. | Protein Accession No. | Amino Acid Sequence (N→C) | SEQ ID NO: |
|---|---|---|---|---|
| Interleukin | NM_172140. | NP_742152. | MAAAWTVVLVTLVLGLAVAG | 1 |
| 29 | 2 | 1 | PVPTSKPTTTGKGCHIGRFKS LSPQELASFKKARDALEESLK LKNWSCSSPVFPGNWDLRLL QVRERPVALEAELALTLKVLE AAAGPALEDVLDQPLHTLHHI LSQLQACIQPQPTAGPRPRG RLHHWLHRLQEAPKKESAG CLEASVTFNLFRLLTRDLKYV ADGNLCLRTSTHPEST | |
| Interleukin 28A | NM_172138. 2 | NP_742150. 1 | MKLDMTGDCTPVLVLMAAVL TVTGAVPVARLHGALPDARG CHIAQFKSLSPQELQAFKRAK DALEESLLLKDCRCHSRLFPR TWDLRQLQVRERPMALEAEL ALTLKVLEATADTDPALVDVL DQPLHTLHHILSQFRACIQPQ PTAGPRTRGRLHHWLYRLQ EAPKKESPGCLEASVTFNLF RLLTRDLNCVASGDLCV | 2 |
| Interleukin 28B | NM_172139. 4 | NP_742151. 2 | MTGDCMPVLVLMAAVLTVTG AVPVARLRGALPDARGCHIA QFKSLSPQELQAFKRAKDAL EESLLLKDCKCRSRLFPRTW DLRQLQVRERPVALEAELALT LKVLEATADTDPALGDVLDQ PLHTLHHILSQLRACIQPQPT AGPRTRGRLHHWLHRLQEA PKKESPGCLEASVTFNLFRLL TRDLNCVASGDLCV | 3 |

### Agent Capable of Detecting Biomarker

As used herein, the term "detection of a biomarker" is intended to determine whether a biomarker is present or absent in a sample and/or to measure the level (concentration) of a biomarker.

In the present disclosure, an agent capable of detecting the biomarker, that is, at least one selected from the group consisting of interleukin 28, interleukin 29, and genes coding therefor may be any one small molecular weight chemical, protein, or nucleic acid molecule if it can bind to the biomarker.

In an embodiment, when the biomarker is at least one protein selected from the group consisting of interleukin 28 and interleukin 29, the agent capable of detecting the biomarker may be at least one selected from the group consisting of proteins (e.g., antibodies, antigen-binding fragments thereof, antibody analogs bearing the antigen-binding fragment, receptors, etc.), peptides, nucleic acids (e.g., polynucleotides, oligonucleotides, etc.), and small molecule chemicals, which all bind to the biomarker, but are not limited thereto.

In another embodiment, when the biomarker is at least one gene (full-length DNA, cDNA, or mRNA) selected from the group consisting of an interleukin 28-encoding gene and an interleukin 29-encoding gene, the agent capable of detecting the biomarker may be at least one selected from the group consisting of nucleic acid molecules (oligonucleotides, polynucleotides, and the like; e.g., primers, probes, aptamers, antisense oligonucleotides, etc.) and small-molecule chemicals, which can all associate (or hybridize) with the gene, but with no limitations thereto.

The agent capable of detecting the biomarker may be labeled with or without a general label such as a fluorophore, a chromophore, a luminophore, an isotope, a heavy metal, etc.

In an embodiment, when the biomarker is at least one protein selected from the group consisting of interleukin 28 and interleukin 29, the detection of the biomarker may be achieved using a typical protein detection (or measurement or analysis) method such as enzymatic reactions, detection of fluorescence, luminescence, and/or radiation. Examples of the detection method comprise, but are not limited to, Immunochromatography, immunohistochemical staining, enzyme-linked immunosorbent assay (ELISA), radioimmunoassay (RIA), enzyme immunoassay (EIA), fluorescence immunoassay (FIA), luminescence immunoassay (LIA), Western blotting, microarray, and flow cytometry.

In another embodiment, when the biomarker is at least one gene (full-length DNA, cDNA, or mRNA) selected from the group consisting of an interleukin 28-encoding gene and an interleukin 29-encoding gene, the detection of the biomarker may be achieved using a typical gene detection (or measurement or analysis) method. For example, the biomarker can be detected (or measured) by a typical genetic analysis method a primer, a probe, an aptamer, or an antisense oligonucleotide that is hybridizable with the gene, specifically, a polymerase chain reaction method (PCR; e.g., qPCR, real-time PCR, real-time qPCR, and so on), FISH (fluorescent in situ hybridization), microarray, etc., but with no limitations thereto. In an embodiment, the primer is designed to detect a gene fragment accounting for a consecutive sequence of 5 to 1000 bp, e.g., 10 to 500 bp, 20 to 200 bp, or 50 to 200 bp, in the nucleotide sequence of the gene (full-length DNA, cDNA, or mRNA) and may be in the form of a pair of primers that comprise nucleotide sequences which are respectively hybridizable with (e.g., complementary to) consecutive sequences of 5 to 100 bp, for example, 5 to 50 bp, 5 to 30 bp, or 10 to 25 bp in the 3'- and 5'-terminal region of the gene fragment. The probe, aptamer, or antisense oligonucleotide may be 5 to 1000 bp, 5 to 500 bp, 5 to 200 bp, 5 to 100 bp, 5 to 50 bp, 5 to 30 bp, or 5 to 25 bp long in total, comprising a nucleotide sequence that is bindable to or hybridizable with (or complementary to) a gene fragment accounting for a consecutive sequence of 5 to 1000 bp, 5 to 500 bp, 5 to 200 bp, 5 to 100 bp, 5 to 50 bp, 5 to 30 bp, or 5 to 25 bp in the gene (full-length DNA, cDNA, or mRNA) coding for interleukin 28 or interleukin 29. As used herein, the term "bindable" means capable of binding to a part or entirety of the gene through a chemical linkage such as a covalent bond and/or physical association. The term "hybridizable" means pertaining to complementarily binding to a specific nucleotide sequence of the gene, with a sequence complementarity of 80% or higher, e.g., 90% or higher, 95% or higher, 98% or higher, 99% or higher, or 100% between the primer, probe or aptamer and the gene fragment.

### Composition and Kit for Diagnosis of Pancreatic Cancer

A composition for diagnosis of pancreatic cancer, provided herein, may contain the aforementioned agent capable of detecting the biomarker.

A kit for diagnosis of pancreatic cancer, provided herein, may comprise the aforementioned agent capable of detecting the biomarker or a composition containing same, and a detecting means thereof. The detecting mean is designed to qualitatively and/or quantitatively analyze the presence or absence or the level of the biomarker detected with the detectable agent. In an embodiment, the detecting means may be selected from among means used for protein and/or gene detection methods described in the foregoing.

In an embodiment, the kit may be an RT-PCR kit, a DNA chip kit, an ELISA kit, a protein chip kit, a rapid kit, or an MRM (multiple reaction monitoring) kit, but is not limited thereto.

### Method for Diagnosing Pancreatic Cancer

The method for diagnosing pancreatic cancer (or method for providing information for diagnosis), provided herein, may comprise a step of detecting a biomarker, which is at least one selected from the group consisting of interleukin 28, interleukin 29, and genes coding therefor, in a blood sample isolated from a subject.

In the method, the step of detecting a biomarker may be a step adapted to perform either or both of identifying the presence or absence of a biomarker in a sample and measuring a level (concentration) of a biomarker in a sample. In the method, when the biomarker is present in the sample or is measured at a higher level in the sample than a reference sample (control), the sample or a subject from which the sample is originated may be diagnosed (or identified or determined) as a pancreatic cancer patient.

The method for diagnosing pancreatic cancer or for providing information for diagnosis of pancreatic cancer, provided herein, may comprise the steps of:
(a) detecting a biomarker in a blood sample isolated from a subject, wherein the biomarker is at least one selected from the group consisting of interleukin 28, interleukin 29, and genes coding therefor; and
(b) diagnosing (or identifying or determining) the sample or a subject from which the sample is originated as a pancreatic cancer patient, when the biomarker is present in the blood sample or measured at a higher level in the blood sample than a reference sample (control).

In addition, when designed to measure a level of a biomarker in the sample, the method may further comprise the steps of:
(a) measuring a level of the biomarker in a blood sample isolated from a subject, wherein the biomarker is at least one selected from the group consisting of interleukin 28, interleukin 29, and genes coding therefor; and
(b)
   (i) comparing the measured level of the biomarker in the blood sample with that of the biomarker in a reference sample,
   (ii) diagnosing (or identifying or determining) the sample or a subject from which the sample is originated as a pancreatic cancer patient when the level of the biomarker is higher in the sample than the reference sample, or
   (iii) both of steps (i) and (ii). Optionally, the method may further comprise a step of measuring a level of the biomarker in the reference sample prior to step (b) (step (i) and/or (ii)).

As used herein, the "level of the biomarker is higher" in the sample than the reference sample may mean that the concentration or the biomarker or the number of cells expressing the biomarker in the sample is greater by about 1.1 fold or more, about 1.2 fold or more, about 1.3 fold or more, about 1.5 fold or more, about 1.8 fold or more, about 2 fold or more, about 2.5 fold or more, about 3 fold or more, about 3.5 fold or more, about 4 fold or more, about 4.5 fold or more, or about 5 fold or more than in the reference sample.

The method for diagnosing pancreatic cancer or for providing information for diagnosis of pancreatic cancer, provided herein, may further comprise a step of
(c) treating pancreatic cancer in a subject who has been diagnosed as a pancreatic cancer patient, after the detecting step, the comparing step, or diagnosing step.

The treatment of pancreatic cancer may mean chemotherapy such as administration of a therapeutic agent for pancreatic cancer (e.g., anticancer agent: 5-fluorouracil, gemcitabine, tarceva (erlotinib), antibody, etc.), radiotherapy, surgical operation, or a combination thereof.

### Blood Sample

The sample or blood sample of a subject to be diagnosed, to which the pancreatic cancer diagnosis composition, kit, and method, provided herein, is applicable, may include a liquid biopsy, for example, blood, serum, plasma, and/or cells separated therefrom, which are all obtained (or isolated or derived) from the subject. In an embodiment, the sample may comprise a buffy coat separated from the subject to be diagnosed.

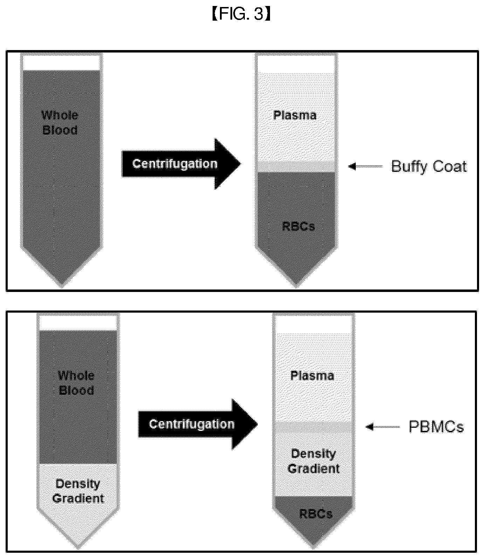

As used herein, the term "buffy coat" refers to a whole white blood cell layer formed between the upper plasma layer and the lower red blood cell layer following centrifugation and is a mix of blood components (e.g., monocytes, granulocytes, lymphocytes, etc.), except for plasma and erythrocytes, with an explicitly different concept from peripheral blood mononuclear cells (PBMCs) (see FIG. 3). The blood sample used herein may be a blood-derived buffy coat, but may be not a sample composed only of peripheral blood mononuclear cells. In an embodiment, the buffy coat may be obtained from the medium layer which is formed together with the upper plasma layer and the lower red blood cell layer following centrifugation under at least one selected from the following conditions (1) to (3):
(1) Temperature: 2 to 30°C, 2 to 28°C, 2 to 25°C, 2 to 23°C, 2 to 20°C, 2 to 18°C, 2 to 15°C, 2 to 13°C, 2 to 10°C, 2 to 8°C, 2 to 6°C, 2 to 5°C, 2 to 4°C, 3 to 30°C, 3 to 28°C, 3 to 25°C, 3 to 23°C, 3 to 20°C, 3 to 18°C, 3 to 15°C, 3 to 13°C, 3 to 10°C, 3 to 8°C, 3 to 6°C, 3 to 5°C, 3 to 4°C, 4 to 30°C, 4 to 28°C, 4 to 25°C, 4 to 23°C, 4 to 20°C, 4 to 18°C, 4 to 15°C, 4 to 13°C, 4 to 10°C, 4 to 8°C, 4 to 6°C, 4 to 5°C, 5 to 30°C, 5 to 28°C, 5 to 25°C, 5 to 23°C, 5 to 20°C, 5 to 18°C, 5 to 15°C, 5 to 13°C, 5 to 10°C, 5 to 8°C, 5 to 6°C, 10 to 30°C, 10 to 28°C, 10 to 25°C, 10 to 23°C, 10 to 20°C, 10 to 18°C, 10 to 15°C, 10 to 13°C, 15 to 30°C, 15 to 28°C, 15 to 25°C, 15 to 23°C, 15 to 20°C, 15 to 18°C, 20 to 30°C, 20 to 28°C, 20 to 25°C, or 20 to 23°C;
(2) Speed: 300g to 2000g, 300g to 1800g, 300g to 1500g, 300g to 1300g, 300g to 1000g, 500g to 2000g, 500g to 1800g, 500g to 1500g, 500g to 1300g, 500g to 1000g, 600g to 1000g, 700g to 1000g, 800g to 1000g, 500g to 900g, 600g to 900g, 700g to 900g, 800g to 900g, 500g to 800g, 600g to 800g, or 700g to 800g; and
(3) Duration time: 5 to 20 min, 7 to 20 min, 9 to 20 min, 5 to 15 min, 7 to 15 min, 9 to 15 min, 5 to 12 min, 7 to 12 min, or 9 to 12 min.

In the present disclosure, the subject to be diagnosed may be selected from mammals including primates such as humans, monkeys, and the like, and rodents such as rats, etc., and may be an individual in need of diagnosis for pancreatic cancer.

In the present disclosure, the reference sample is a sample obtained (or isolated or derived) from a normal (e.g., not suffering from pancreatic cancer) individual. For instance, the normal individual may be homologous to the subject to be diagnosed and is selected from mammals including primates such as humans, monkeys, and the like, and rodents such as rats, etc. The reference sample may include blood, serum, plasma, and/or cells separated therefrom, which are all obtained (or isolated or derived) from the reference subject. In an embodiment, the sample may comprise a buffy coat.

### Screening Therapeutic Agent for Pancreatic Cancer

Provided in another aspect is a method for screening a therapeutic drug candidate for pancreatic cancer, in which a level of the biomarker is measured in the presence of candidate compounds.

More specifically, the screening method may comprise the steps of:
contacting a biological sample with a candidate compound;
measuring a level of a biomarker in the biological sample, the biomarker being at least one selected from the group consisting of interleukin 28, interleukin 29, and genes coding therefor; and
comparing the level of the biomarker in the biological sample that has been contacted with the candidate compound with a level of the biomarker in the biological sample that has not been contacted with the candidate compound.

The comparing step may be carried out by measuring levels of the biomarker in the same biological sample before and after contact (treatment) with a candidate compound, followed by comparison therebetween or by contacting only a part of a biological sample with a candidate compound, measuring levels of the biomarker in the parts of the biological sample which have been and not been contacted with the candidate compound, respectively, and followed by comparison therebetween.

When the biological sample contacted with the candidate compound has a lower level of the biomarker than the biological sample contacted without the candidate compound, that is, when the candidate compound lowers the level of the biomarker or inhibits the expression of the biomarker in the biological sample, the candidate compound may be determined as a promising candidate for a drug for prevention and/or treatment of pancreatic cancer.

The biological sample may comprise a biopsy, for example, blood, serum, plasma, and/or cells separated therefrom, which are all obtained (or isolated or derived) from a pancreatic cancer patient. Specifically, the biological sample may comprise a buffy coat.

The candidate compound may be selected from the group consisting of various compounds, for example, low-molecular weight compounds, proteins, polypeptides, oligopeptides, polynucleotides, oligonucleotides, and extracts from plants or animals.

Measuring a level of a biomarker in a biological sample may be carried out by using a typical detection means for quantifying a gene or a protein and/or evaluating the measurements. Concrete measurement means are described in the foregoing.

### Advantageous Effects

With the ability to early diagnose pancreatic cancer at high accuracy in a simple and non-invasive manner only with blood derived from a subject, the present disclosure can solve the problems, encountered by conventional methods, of needing histological examination and having difficulty in early diagnosis.

### Description of Drawings

FIG. 1 is a graph showing expression level differences of IL-29 in buffy coat and PBMC samples obtained from pancreatic cancer patients and normal persons.
FIG. 2 is a graph showing expression level differences of IL-28 in buffy coat and PBMC samples obtained from pancreatic cancer patients and normal persons.
FIG. 3 is a schematic view illustrating a process of preparing a buffy coat sample in comparison with a PBMC sample.
FIG. 4 shows graphs of expression levels of IL-28 (upper) and IL-29 (lower) in buffy coat and PBMC samples obtained from the same pancreatic cancer patients.
FIG. 5a is a graph showing expression levels of IL-28 in buffy coat and PBMC samples obtained from pancreatic cancer patients in comparison with that that in PBMC samples from normal persons.
FIG. 5b is a graph showing expression levels of IL-28 in buffy coat and PBMC samples obtained from pancreatic cancer patients in comparison with that that in buffy coat samples from normal persons.
FIG. 6a is a graph showing expression levels of IL-29 in buffy coat and PBMC samples obtained from pancreatic cancer patients in comparison with that that in PBMC samples from normal persons.
FIG. 6b is a graph showing expression levels of IL-29 in buffy coat and PBMC samples obtained from pancreatic cancer patients in comparison with that that in buffy coat samples from normal persons.
FIG. 7a is a graph showing expression levels of IL-28 in buffy coat and PBMC samples obtained from pancreatic cancer patients in comparison with that that in samples (average value of IL-28 expression levels in PBMC and buffy coat samples) from normal persons.
FIG. 7b is a graph showing expression levels of IL-29 in buffy coat and PBMC samples obtained from pancreatic cancer patients in comparison with that that in samples (average value of IL-29 expression levels in PBMC and buffy coat samples) from normal persons.

### Mode for Invention

A better understanding of the present disclosure may be obtained through the following examples which are set forth to illustrate, but are not to be construed as limiting the present disclosure.

### EXAMPLE 1: Sample Preparation

### 1.1. Buffy coat sample preparation

An examination was made of expression changes of pancreatic cancer biomarkers between pancreatic cancer samples and normal samples. In this regard, mRNA expression of IL-29 and IL-28 was analyzed in buffy coats separated from blood of pancreatic cancer patients. For comparison, the same experiment was conducted with the blood-derived buffy coats from healthy persons (hereinafter referred to as normal subjects).

First, 5 cc of blood from each of pancreatic cancer patients and normal subjects (normal control) was sampled into an EDTA tube and stored at room temperature. The blood-containing EDTA tube was centrifuged at 800 g and 4°C for 10 min. In the tube, a plasma, a buffy coat, and red blood cells were layered in the order from the top. After removal of the plasma, the buffy coat was recovered in an amount of 250 µl and stored at -80°C.

Total RNA was extracted from 250 µl of the deep-frozen buffy coat using NucleoSpin^{®} RNA Blood kit (MACHEREY-NAGEL) according to the recommended protocol. From 1 µg of the RNA, cDNA was synthesized. For cDNA synthesis, GoScript^{™} Reverse Transcription system kit (Promega) was used.

### 1.2. Peripheral blood mononuclear cell (PBMC) sample preparation

An examination was made of expression changes of pancreatic cancer biomarkers between pancreatic cancer samples and normal samples. In this regard, mRNA expression of IL-29 and IL-28 (IL-28A) was analyzed in peripheral blood mononuclear cells from blood of pancreatic cancer patients. For comparison, the same experiment was conducted with the peripheral blood mononuclear cells from healthy persons (hereinafter referred to as normal subjects).

First, 5 cc of blood from each of pancreatic cancer patients and normal subjects (normal control) was sampled into an EDTA tube and stored at room temperature. When blood is high in viscosity, it is difficult to separate mononuclear cells from the blood. Thus, the blood sample was diluted at a ratio of 1:1 with 1X PBS. The lymphocyte separation medium (Lymphosep^{™}, L0560-500, Biowest) was prepared as a density gradient medium in a 15-ml conical tube, followed by adding the blood sample on Ficoll, with care to prevent mingling with the separation medium. Centrifugation at 800g for 30 min with minimum acceleration and deceleration resulted in separating the blood into plasma, peripheral blood mononuclear cells (PBMC), Lymphosep, granulocytes, and erythrocytes in the density-increasing order from the top to the bottom in the tube. The medium white cell layer (monocytes) was recovered. The cells were added with PBS, centrifuged at 400g for 3 min, and washed twice before use. The residual cells were stored at -80°C.

The PBMC thus prepared was added with 1 ml of Trizol to suspend the adherent cells. After 200 µl of chloroform, centrifugation was conducted at 12,000g for 15 min. Only the transparent uppermost supernatant was picked out and mixed with an equal volume of isopropanol before centrifugation at 12,000g for 15 min. After the supernatant was discarded, the pellet was washed with 1 ml of 75% ethanol by centrifugation at 12,000g for 10 min. The supernatant was discarded and the pellet was sufficiently dried for 1 hour. The RNA pellet was added with 30 µl of nuclease-free water before RNA quantitation. For cDNA synthesis, 1 µg of RNA was used. cDNA synthesis was carried out using GoScript^{™} Reverse Transcription system kit (Promega).

### EXAMPLE 2. Real-time qPCR

Gene expression in the samples prepared in Example 1 was measured by real-time qPCR. The real-time qPCR was carried out in a probe-based multiplex PCR assay. For multiplex PCR, respective probes for the markers IL-29 (GenBank Accession No. NM_172140.2) and IL-28A (GenBank Accession No. NM_172138.2; hereinafter referred to as " IL-28") were labeled with FAM dye while GAPDH for use as an internal reference gene was labeled with HEX dye. Primers and probes were purchased from IDT (Integrated DNA Technologies, Inc.).

To identify the expression of each gene in the samples, a reaction mix including GoTaq^{®} Probe qPCR Master Mix (Promega) was prepared in a final volume of 20 µl according to the protocol. Gene expression assay was performed using QuantStudio 3 and 5 Real-Time PCR system instrument (Applied Biosystems) under the standard cycling condition provided by the software of the instrument. Real-time qPCR results were expressed as ΔCt values, which were differences between Ct values, that is, mRNA expression levels of the markers IL-29 and IL-28 and the internal reference gene GAPDH. All data for comparison of mRNA expression were analyzed in terms of ΔCt.

Nucleotide sequences (5'→3') of the primers and probes used are summarized in Table 2, below.

**TABLE 2**

| | Sequence (5'→3') | SEQ ID NO: |
|---|---|---|
| IL-29_F primer | GGT TCA AAT CTC TGT CAC CAC A | 4 |
| IL-29_R primer | GAA GAC AGG AGA GCT GCA AC | 5 |
| IL-28A_F primer | CAG CCT CAG AGT GTT TCT TCT | 6 |
| IL-28A_R primer | TCC AGT CAC GGT CAG CA | 7 |
| GPC-1_F primer | GTC ATG AAG CTG GTC TAC TG | 8 |
| GPC-1_R primer | AGC CCT TGA GCA CAT TTC | 9 |
| IL-29_Probe | FAM/TCAAGAAGG/ZEN/CCAGGGACGCC/IBFQ | 10 |
| IL-28_Probe | FAM- TCATGTCTA/ZEN/GTTTCATTCCTGATCTCTGGTCT- IBFQ | 11 |

| | | |
|---|---|---|
| (in Table 2, the probes were structured to include 5' FAM dye, internal ZEN Quencher, and 3' Iowa Black^{®} Fluorescent Quencher (IBFQ)) | | |

### EXAMPLE 3: Measurement of Marker Expression in Each Sample

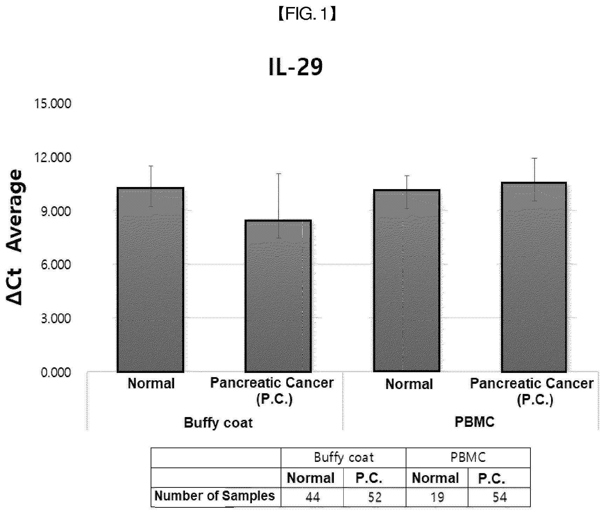

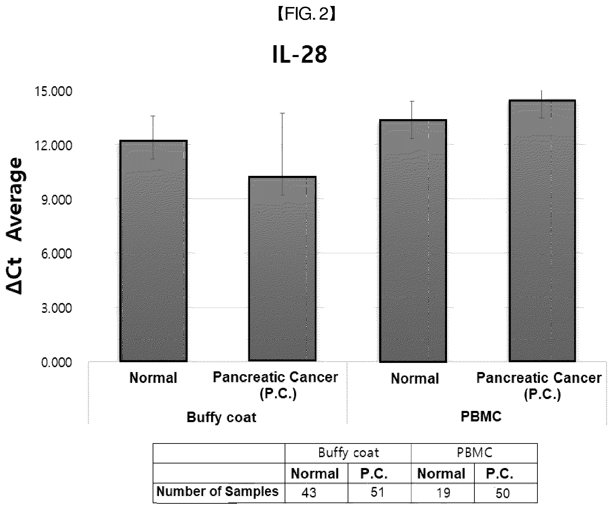

Results of the real-time qPCR performed in Example 2 are depicted in FIGS. 1 and 2.

FIG. 1 is a graph showing expression level differences of IL-29 in buffy coat samples obtained from 52 pancreatic cancer patients and 44 normal persons and in PBMC samples obtained from 54 pancreatic cancer patients and 19 normal persons. As can be seen in FIG. 1, the average ΔCt value of IL-29 was significantly reduced in the buffy coat samples from the pancreatic cancer patients, compared to the normal persons. In contrast, the average ΔCt value of IL-29 in the PBMC samples were rather increased for the pancreatic cancer patients, compared to the normal persons, with an insignificant difference therebetween. From the data, it was identified that the expression level of IL-29 was significantly increased in pancreatic cancer patients, compared to normal persons, and the expression pattern of IL-29 in pancreatic cancer patients distinctively differed from buffy coat samples to PBMC samples.

FIG. 2 is a graph showing expression level differences of IL-28 in buffy coat samples obtained from 51 pancreatic cancer patients and 43 normal persons and in PBMC samples obtained from 50 pancreatic cancer patients and 19 normal persons. As can be seen in FIG. 2, the average ΔCt value of IL-28 was significantly reduced in the buffy coat samples from the pancreatic cancer patients, compared to the normal persons. In contrast, the average ΔCt value of IL-28 in the PBMC samples were rather increased for the pancreatic cancer patients, compared to the normal persons, with an insignificant difference therebetween. From the data, it was identified that the expression level of IL-28 was significantly increased in pancreatic cancer patients, compared to normal persons, and the expression pattern of IL-28 in pancreatic cancer patients distinctively differed from buffy coat samples to PBMC samples.

Taken together, the data indicate that IL-29 and IL-28 are both useful as markers for pancreatic cancer due to their significant difference in expression level between pancreatic cancer patients and normal persons and that when the markers are used, more reliable results could be obtained from a buffy coat sample than a PBMC sample. In addition, a greater difference between pancreatic cancer patients and normal persons was observed for IL-28 than IL-29.

### EXAMPLE 4: Comparison between Buffy Coat and PBMC from Same Person

From the same persons (three pancreatic cancer patients (P.C)), buffy coat samples and PBMC samples were prepared referring to Examples 1.1 and 1.2, respectively. The prepared buffy coat and PBMC samples were measured for mRNA expression levels (ΔCt) of IL-29 and IL-28 in the same manner as in Example 2.

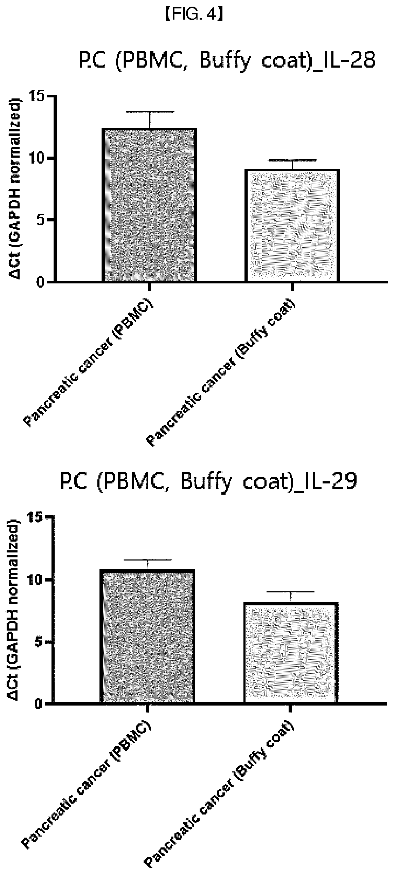

Average mRNA expression levels of IL-29 and IL-28 are depicted in FIG. 4 (No. of samples: three buffy coat samples; three PBMC samples). As shown in Figure 4, the mRNA expression levels (ΔCt) of IL-29 and IL-28 were significantly higher in the buffy coat samples, compared to the PBMC samples,

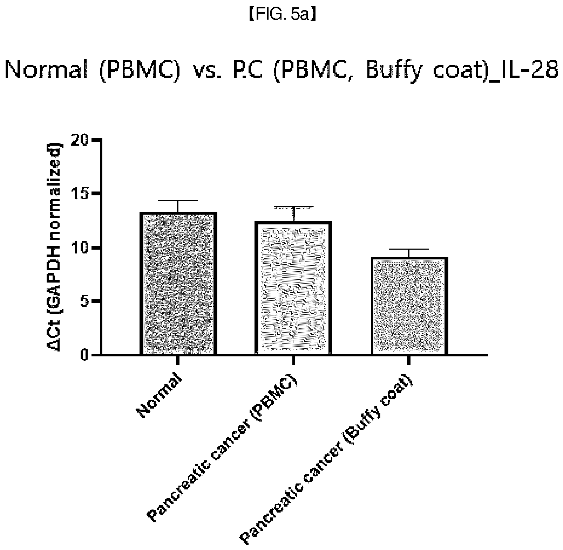

Comparison of mRNA expression levels in buffy coat samples and PBMC samples from same persons were made between pancreatic cancer patients and normal persons and the results are depicted in FIGS. 5a, 5b, 6a, 6b, 7a, and 7b (all expressed as average values).

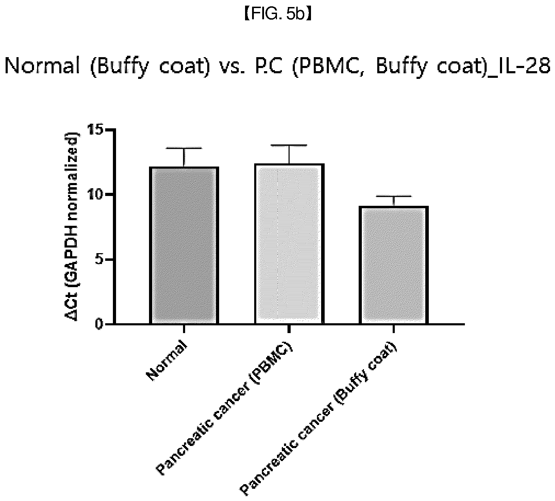

FIG. 5a shows IL-28 expression levels in buffy coat and PBMC samples from pancreatic cancer patients in comparison with that in the PBMC samples from normal persons; and FIG. 5b shows IL-28 expression levels in buffy coat and PBMC samples from pancreatic cancer patients in comparison with that in the buffy coat samples from normal persons (no. of samples: 43 normal person buffy coat samples; 3 pancreatic cancer buffy coat samples; 19 normal person PBMC samples; and 3 pancreatic cancer PBMC samples).

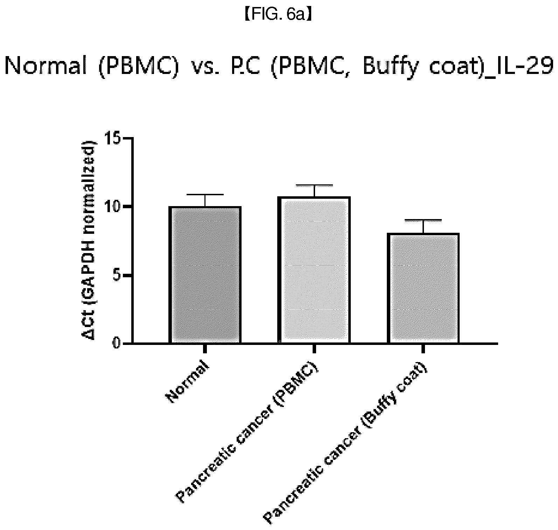

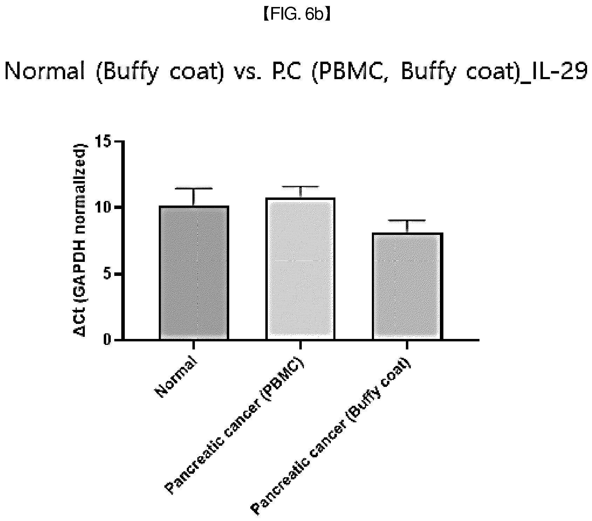

FIG. 6a shows IL-29 expression levels in buffy coat and PBMC samples from pancreatic cancer patients in comparison with that in the PBMC samples from normal persons; and FIG. 6b shows IL-29 expression levels in buffy coat and PBMC samples from pancreatic cancer patients in comparison with that in the buffy coat samples from normal persons (no. of samples: 44 normal person buffy coat samples; 3 pancreatic cancer buffy coat samples; 19 normal person PBMC samples; and 3 pancreatic cancer PBMC samples).

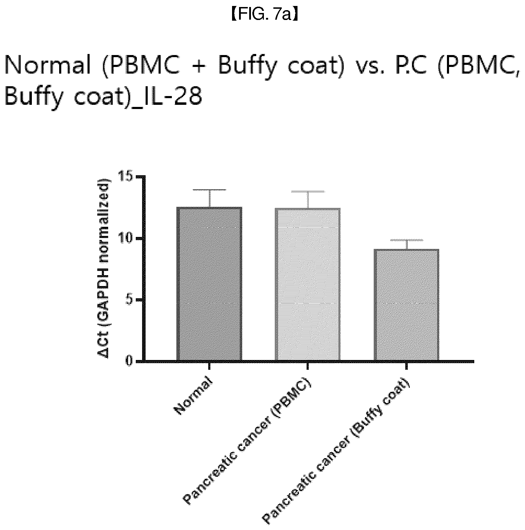

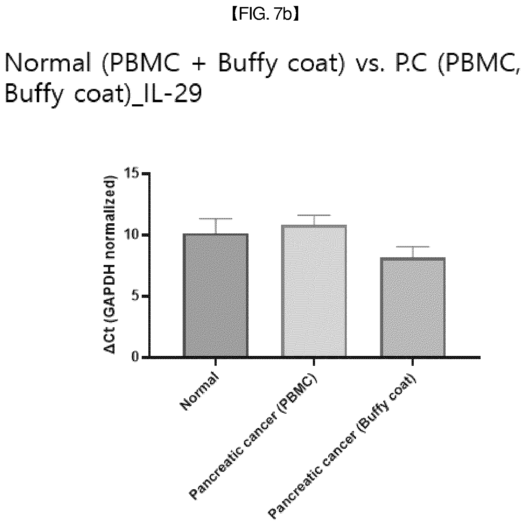

FIG. 7a shows IL-28 expression levels in buffy coat and PBMC samples from pancreatic cancer patients in comparison with that in the samples from normal persons (average value of integrated IL-28 expression levels in both PBMC and buffy coat samples); and FIG. 7b shows IL-29 expression levels in buffy coat and PBMC samples from pancreatic cancer patients in comparison with that in the samples from normal persons (average value of integrated IL-29 expression levels in both PBMC and buffy coat samples) (no. of samples: 43 and 44 normal person buffy coat samples (for IL-28 and IL-29, respectively); 3 pancreatic cancer buffy coat samples; 19 normal person PBMC samples; and 3 pancreatic cancer PBMC samples).

As shown in FIGS. 5a, 5b, 6a, 6b, 7a, and 7b, no significant differences of IL-28 and IL-29 expression levels in PBMC samples were not observed between normal persons and pancreatic cancer patients. In contrast, a remarkable difference of IL-28 and/or IL-29 expression level in the buffy coat samples was detected between normal persons and pancreatic cancer patients. These results imply that that pancreatic cancer patients can be diagnosed more accurately and efficiently by measuring the levels of IL-28 and/or IL-29 in buffy coat samples.

## Claims

1. A composition for diagnosis of pancreatic cancer, wherein the composition comprises an agent capable of detecting at least one selected from the group consisting of interleukin 28, interleukin 29, and genes coding therefor, and is applied to a blood-derived buffy coat.

2. The composition of claim 1, wherein the buffy coat is a whole white blood cell layer formed between the upper plasma layer and the lower red blood cell layer following centrifugation and is obtained by forming an upper plasma layer, a medium buffy coat layer, and a lower red blood cell layer upon centrifugation under at least one selected from the following conditions (1) to (3) and isolating the medium buffy coat layer:
(1) temperature: 2 to 30°C;
(2) speed: 300g to 2000g; and
(3) duration time: 5 to 20 minutes.

3. The composition of claim 1, wherein the buffy coat is obtained by forming an upper plasma layer, a medium buffy coat layer, and a lower red blood cell layer upon centrifugation under at least one selected from the following conditions (1) to (3) and isolating the medium buffy coat layer:
(1) temperature: 4 to 25°C;
(2) speed: 500g to 1800g; and
(3) duration time: 7 to 20 minutes.

4. The composition of any one of claims 1 to 3, wherein the detecting agent is at least one selected from consisting of low-molecular weight compounds, proteins, peptides, and nucleic acids, which are all able to bind to at least one selected from the group consisting of interleukin 28, interleukin 29, and genes coding therefor.

5. A kit for diagnosis of pancreatic cancer, comprising the composition of any one of claims 1 to 3.

6. The kit of claim 5, wherein the kit is applied to a blood-derived buffy coat.

7. A method for providing information for diagnosis of pancreatic cancer, the method comprising a step of detecting at least one selected from the group consisting of interleukin 28, interleukin 29, and genes coding therefor in a blood sample isolated from a subject, wherein the blood sample is a blood-derived buffy coat.

8. The method of claim 7, further comprising, after the detecting step, a step of determining the subject to be a pancreatic cancer patient when the biomarker is present in the blood sample or when the level of the biomarker is higher in the sample than a normal reference sample.

9. The method of claim 7, wherein the buffy coat is a whole white blood cell layer formed between the upper plasma layer and the lower red blood cell layer following centrifugation and is obtained by forming an upper plasma layer, a medium buffy coat layer, and a lower red blood cell layer upon centrifugation under at least one selected from the following conditions (1) to (3) and isolating the medium buffy coat layer:
(1) temperature: 2 to 30°C;
(2) speed: 300g to 2000g; and
(3) duration time: 5 to 20 minutes.

10. The method of claim 7, wherein the buffy coat is obtained by forming an upper plasma layer, a medium buffy coat layer, and a lower red blood cell layer upon centrifugation under at least one selected from the following conditions (1) to (3) and isolating the medium buffy coat layer:
(1) temperature: 4 to 25°C;
(2) speed: 500g to 1800g; and
(3) duration time: 7 to 20 minutes.
